# EUROPEAN PATENT APPLICATION

(11) **EP 1 367 053 A1**
(43) Date of publication of application: **03.12.2003**
(21) Application number: 02701575.9
(22) Date of filing: 27.02.2002
(51) Int. Cl.: C07D 213/75, C07D 317/34, C07C 231/02, C07C 231/18, C07C 235/16, C07C 215/16, C07C 213/02, C07C 231/10

(54) **PROCESS FOR PRODUCING OPTICALLY ACTIVE N-ARYL-1-AMINO-2-PROPANOL DERIVATIVES**

(30) Priority: 27.02.2001 JP 2001051449; 25.10.2001 JP 2001328345
(71) Applicant: KANEKA CORPORATION, Osaka-shi, Osaka 530-8288 (JP)
(72) Inventor: TAOKA, Naoaki c/o KANEKA CORPORATION, Takasago-shi, Hyogo 676-0027 (JP); YASOHARA, Yoshihiko c/o KANEKA CORPORATION, Takasago-shi, Hyogo 676-0027 (JP); YAO, Takeshi c/o KANEKA CORPORATION, Settsu-shi, Osaka 566-0072 (JP); MAEHARA, Katsuji c/o KANEKA CORPORATION, Settsu-shi, Osaka 566-0072 (JP); UEDA, Yasuyoshi c/o KANEKA CORPORATION, Takasago-shi, Hyogo 676-0027 (JP)
(74) Representative: HOFFMANN - EITLE
(86) International application number: JP0201767
(87) International publication number: WO02070482

(57) **Abstract**

The present invention has an object to provide a process for easily producing optically active N-aryl-1-amino-2-propanol derivatives which are of value as pharmaceutical intermediates from inexpensive starting materials.

The above object can be attained by producing an optically active N-aryl-1-amino-2-propanol derivative by the process which comprises reacting an optically active lactate derivative or an optically active lactic acid acetal derivative, which are available at low cost, with an arylamine derivative to give an optically active N-aryllactamide derivative and treating it with a reducing agent.

## Description

### TECHNICAL FIELD

The present invention relates to a process for producing optically active N-aryl-1-amino-2-propanol derivatives which are of value as pharmaceutical intermediates. More particularly, the invention relates to a process for producing an optically active N-aryl-1-amino-2-propanol derivative, particularly (S)-N-(2-pyridyl)-1-amino-2-propanol, which comprises synthesizing an optically active N-aryllactamide derivative in an expedient way and reducing the same.

### BACKGROUND ART

The following processes are known for the production of optically active N-aryl-1-amino-2-propanol derivatives.

### 1) The process for synthesizing

(S)-N-(2-pyridyl)-1-amino-2-propanol which comprises reacting (S)-1-amino-2-propanol with 2-chloropyridine in the presence of potassium t-butoxide and heating the reaction product in the presence of a catalyst amount of p-toluenesulfonic acid (WO 95/33743).

### 2) The process for synthesizing

4-(2(S)-hydroxypropylamino)-3-methylsulfonylbenzoic acid which comprises reacting (S)-1-amino-2-propanol with 4-fluoro-3-methylsulfonylbenzoic acid under heating in the presence of diisopropylethylamine (Japanese Kokai Publication Hei-06-234727).

However, the optically active 1-amino-2-propanol, the starting material, is very expensive and not readily available in large quantities so that these processes cannot be easily practiced on a commercial scale.

Meanwhile, the following process is known for the production of optically active N-aryllactamide derivatives.

The process which comprises reacting (S)-5-methyl-1,3-dioxolane-2,4-dione with aniline to synthesize (S)-2-hydroxy-N-phenylpropanamide (Heterocycles, 1989, 29, 5, 975-978).

When an optically active N-aryllactamide derivative is synthesized from an optically active lactic acid derivative and the corresponding arylamine derivative, the conditions in routine use for synthesizing optically active N-alkyllactamide derivatives in general does not allow the reaction to proceed appreciably because of the low nucleophilicity of the arylamine derivative (Synthesis, 1986, 1, 60). It is, therefore, necessary to use an activated derivative of (S)-lactic acid, e.g. (S)-5-methyl-1,3-dioxolane-2,4-dione, as the starting material as in the production example described above. However, synthesis of (S)-5-methyl-1,3-dioxolane-2,4-dione requires use of phosgene which is an extremely toxic and, therefore, much remains to be improved for commercial exploitation of the process.

As an example of reducing the amido group of an optically active lactamide derivative to an amino group, a process is known for synthesizing (S)-1-amino-2-propanol which comprises reducing (S) -lactamide with a borane-THF (tetrahydrofuran) (J. Org. Chem., 1995, 66, 16, 5157). However, no report is available on the process for reducing optically active N-aryllactamides.

Furthermore, the optically active N-aryllactamide derivative, particularly an optically active N-pyridyllactamide derivative, which serves as a key intermediate in the production of optically active N-aryl-1-amino-2-propanol derivatives is a novel compound that has not been described in the literature.

### SUMMARY OF THE INVENTION

In the above state of the art, the present invention has for its object to provide a production technology by which optically active N-aryl-1-amino-2-propanol derivatives and optically active N-aryllactamide derivatives, both of which are of value as pharmaceutical intermediates, particularly optically active N-pyridyl-1-amino-2-propanol derivatives and optically active N-pyridyllactamide derivatives, especially an optically active N-(2-pyridyl)-1-amino-2-propanol and an optically active N-(2-pyridyl)lactamide, can be produced easily from inexpensive, readily available starting materials.

To accomplish the above object, the inventors of the present invention conducted intensive investigations and, as a result, found that an optically active N-aryl-1-amino-2-propanol derivative can be expediently produced by the process which comprises reacting an optically active lactate derivative, which is available at low cost, or an optically active lactic acid acetal derivative, which can be easily synthesized from an inexpensive, readily available optically active lactic acid, with an arylamine derivative to give an optically active N-aryllactamide derivative and treating it with a reducing agent.

It was also found that in order to obtain an optically active N-aryl-1-amino-2-propanol derivative of high quality with good efficiency, this optically active N-aryl-1-amino-2-propanol derivative is preferably purified by extraction and distillation in accordance with a certain protocol.

In connection with the production of an optically active N-pyridyl-l-amino-2-propanol derivative according to the above technology, it was also found that in order to obtain the optically active N-pyridyl-1-amino-2-propanol derivative of high quality (high chemical purity, high optical purity), it is preferable that reaction mixture containing the precursor optically active N-pyridyllactamide derivative be subjected to washing and extraction in accordance with a certain protocol and this amide derivative be then caused to be purified by crystallization from an organic solvent.

The optically active N-pyridyllactamide derivative (a preferred example of which is the optically active N-(2-pyridyl)lactamide derivative) mentioned above is a novel compound which had not been described in the literature but was found by the present inventors to be useful for the production of said optically active N-pyridyl-1-amino-2-propanol derivative (preferably optically active N-(2-pyridyl)-1-amino-2-propanol derivative).

The present invention, therefore, provides an optically active N-pyridyllactamide derivative
which is represented by the general formula (4a): 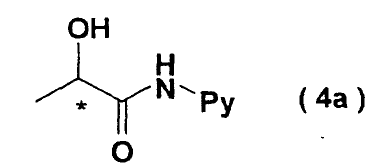 (in the formula, Py represents 2-pyridyl group which may be substituted, 3-pyridyl group which may be substituted, or 4-pyridyl group which may be substituted; * represents an asymmetric carbon atom).

The present invention further provides a process for producing an optically active N-aryllactamide derivative of the general formula (4): 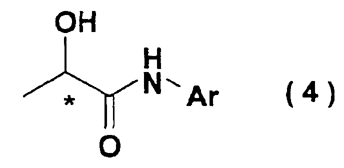 (in the formula, Ar represents an aromatic group which may be substituted; * represents an asymmetric carbon atom),
which comprises reacting an optically active lactate derivative of the general formula (1): 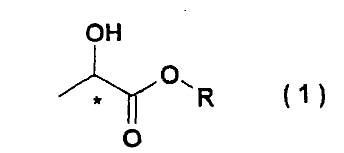 (in the formula, R represents a C₁₋₁₀ alkyl group; * is as defined above) or an optically active lactic acid acetal derivative of the general formula (2): 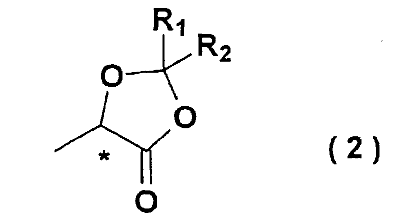 (in the formula, R₁ and R₂ each independently represents hydrogen atom, a C₁₋₁₀ alkyloxy group, or a C₁₋₁₀ alkyl group; * is as defined above) with an arylamine derivative of the general formula (3):

Ar-NH₂ (3)

(in the formula, Ar is as defined above).

The present invention further provides a process for purifying an optically active N-pyridyllactamide derivative,
which comprises fractionating an optically active N-pyridyllactamide derivative of the general formula (4a): 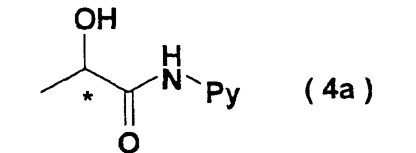 (in the formula, Py represents 2-pyridyl group which may be substituted, 3-pyridyl group which may be substituted, or 4-pyridyl group which may be substituted; * represents an asymmetric carbon atom) containing an optically active lactate derivative of the general formula (1): 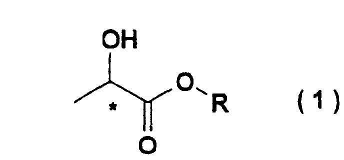 (in the formula, R represents a C₁₋₁₀ alkyl group; * is as defined above) or an optically active lactic acid acetal derivative of the general formula (2): 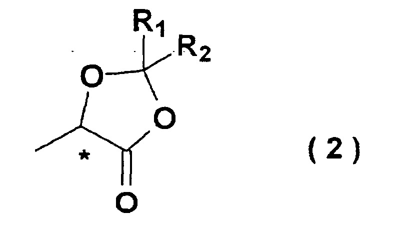 (in the formula, R₁ and R₂ each independently represents hydrogen atom, a C₁₋₁₀ alkyloxy group, or a C₁₋₁₀ alkyl group; * is as defined above) and any byproduct derived therefrom as impurities in a biphasic system comprising water and an organic solvent under an acidic condition with the optically active N-pyridyllactamide derivative (4a) being separated in an aqueous layer and
removing the organic solvent layer.

The present invention further provides a process for purifying an optically active N-pyridyllactamide derivative,
which comprises purifying an optically active N-pyridyllactamide derivative of the general formula (4a): 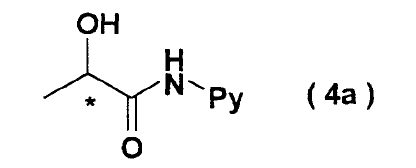 (in the formula, Py is as defined above; * represents an asymmetric carbon atom) containing an aminopyridine derivative of the general formula (3a):

Py-NH₂ (3a)

(in the formula, Py represents 2-pyridyl group which may be substituted, 3-pyridyl group which may be substituted, or 4-pyridyl group which may be substituted) by extracting the optically active N-pyridyllactamide derivative (4a) into an organic layer of a biphasic system comprising water and an organic solvent under a weakly acidic condition with the aminopyridine derivative (3a) being retained in the aqueous layer.

The present invention further provides a process for purifying an optically active N-pyridyllactamide derivative,
which comprises crystallizing an optically active N-pyridyllactamide derivative of the general formula (4a): 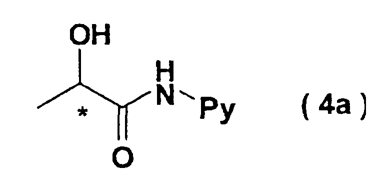 (in the formula, Py represents 2-pyridyl group which may be substituted, 3-pyridyl group which may be substituted, or 4-pyridyl group which may be substituted; * represents an asymmetric carbon atom) containing an impurity from an organic solvent to thereby remove the impurity and obtain the N-pyridyllactamide derivative (4a) as a crystal.

The present invention further provides a process for producing an optically active N-aryl-1-amino-2-propanol derivative of the general formula (5): 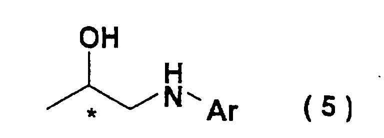 (in the formula, Ar represents an aromatic group which may be substituted; * represents an asymmetric carbon atom),
which comprises reacting either an optically active lactate derivative of the general formula (1): 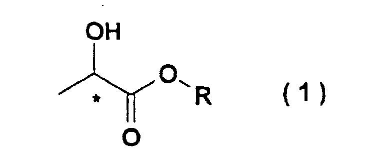 (in the formula, R represents a C₁₋₁₀ alkyl group; * is as defined above) or an optically active lactic acid acetal compound of the general formula (2): 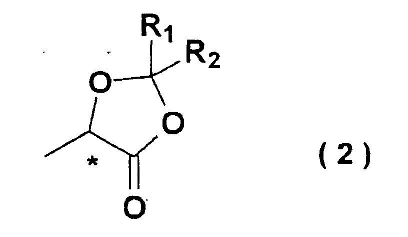 (in the formula, R₁ and R₂ each independently represents hydrogen atom, a C₁₋₁₀ alkyloxy group, or a C₁₋₁₀ alkyl group; * is as defined above) with an arylamine derivative of the general formula (3):

Ar-NH₂ (3)

in the formula, Ar is as defined above
to give an optically active N-aryllactamide derivative of the general formula (4): 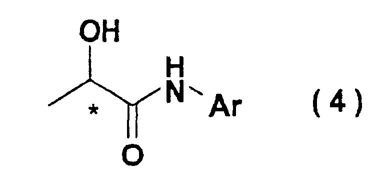 (in the formula, Ar and * are as defined above) and
treating the derivative with a reducing agent.

The present invention further provides a process for producing an optically active N-aryl-1-amino-2-propanol derivative of the general formula (5): 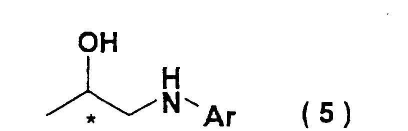 (in the formula, Ar represents an aromatic group which may be substituted; * represents an asymmetric carbon atom),
which comprises reducing an optically active N-aryllactamide derivative of the general formula (4): 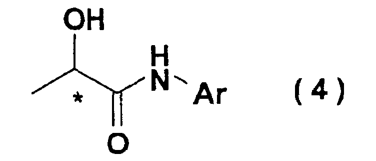 (in the formula, Ar and * are as defined above) with a borane derivative generated in situ by treating a boron hydride complex compound with an acid.

The present invention further provides a process for acquiring an optically active N-aryl-1-amino-2-propanol derivative,
which comprises treating, under an acidic condition, boron-coordinated complex of an optically active N-aryl-1-amino-2-propanol derivative occurring in an optically active N-aryl-1-amino-2-propanol derivative of the general formula (5): 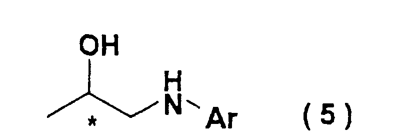 (in the formula, Ar represents an aromatic group which may be substituted; * represents an asymmetric carbon atom) obtainable by reducing an optically active N-aryllactamide derivative of the general formula (4): 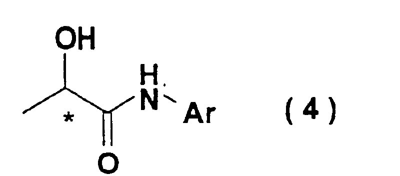 (in the formula, Ar and * are as defined above) with a borane derivative or a boron hydride complex compound, to thereby give the optically active N-aryl-1-amino-2-propanol derivative (5).

The present invention further provides a process for acquiring an optically active N-aryl-1-amino-2-propanol derivative by distillation of an optically active N-aryl-1-amino-2-propanol derivative of the general formula (5) : 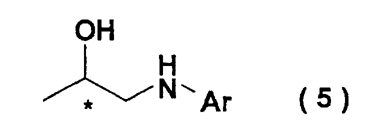 (in the formula, Ar represents an aromatic group which may be substituted; * represents an asymmetric carbon atom) containing an arylamine derivative of the general formula (3):

Ar-NH₂ (3)

(in the formula, Ar is as defined above) as an impurity,
which comprises distilling and recovering the arylamine derivative (3) and, then, distilling and recovering the optically active N-aryl-1-amino-2-propanol derivative (5) from a distillate line inclusive of a condenser which is essentially not contaminated with the arylamine derivative (3).

The present invention further provides a process for acquiring an optically active N-aryl-1-amino-2-propanol derivative of the general formula (5): 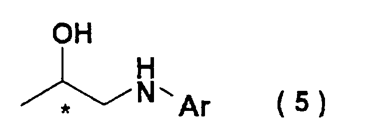 in the formula, Ar represents an aromatic group which may be substituted; * represents an asymmetric carbon atom
by distillation,
which comprises treating a boron component-contaminated extract or concentrate of the optically active N-aryl-1-amino-2-propanol derivative (5) with water and/or an alcohol for decrease or removal of the boron component.

### DETAILED DESCRIPTION OF THE INVENTION

The present invention is now described in detail.

Referring to the above formula (1), R represents an alkyl group containing 1 to 10 carbon atoms. The alkyl group containing 1 to 10 carbon atoms includes methyl, ethyl, n-propyl, isopropyl, n-butyl, isobutyl, sec-butyl, tert-butyl, pentyl, n-hexyl, heptyl, octyl, nonyl, decyl, and so forth. The preferred is a C₁₋₄ alkyl group, more preferred methyl or ethyl.

Referring to the above formula (2), R₁ and R₂ each independently represents hydrogen atom, an alkyloxy group containing 1 to 10 carbon atoms, or an alkyl group containing 1 to 10 carbon atoms.

The alkyloxy group containing 1 to 10 carbon atoms includes methyloxy, ethyloxy, n-propyloxy, isopropyloxy, n-butyloxy, isobutyloxy, sec-butyloxy, tert-butyloxy, pentyloxy, n-hexyloxy, heptyloxy, octyloxy, nonyloxy, decyloxy, and so forth. The preferred is an alkyloxy group containing 1 to 4 carbon atoms, more preferred methyloxy or ethyloxy. The alkyl group containing 1 to 10 carbon atoms includes C₁₋₁₀ alkyl groups similar to those mentioned for R.

R₁ and R₂ may be the same or different. Preferably, both R₁ and R₂ are alkyl groups each containing 1 to 10 carbon atoms, and more preferably both are methyl groups.

Referring to the above formulas (3), (4), and (5), Ar represents an aromatic group which may be substituted. The aromatic group includes aryl groups containing 6 to 12 carbon atoms and heteroaryl groups containing 4 to 10 carbon atoms, among others. Specifically, Ar includes phenyl, naphthyl, biphenyl, pyridyl, furanyl, thienyl, pyrrolyl, oxazolyl, isooxazolyl, pyrazolyl, benzofuranyl, benzothiazolyl, indolyl, and so forth. Ar is preferably pyridyl, i.e. 2-pyridyl, 3-pyridyl, or 4-pyridyl, or phenyl which may be substituted. It is more preferably pyridyl, still more preferably 2-pyridyl.

The substituent which may be present on the above Ar nucleus includes halogen, e.g. fluorine, chlorine, bromine, and iodine; nitro, nitroso, cyano, amino, hydroxylamino, alkylamino containing 1 to 10 carbon atoms, dialkylamino containing 2 to 10 carbon atoms, N-protected amino, azido, trifluoromethyl, carboxyl, formyl, acetyl, benzoyl, hydroxyl, alkyl containing 1 to 10 carbon atoms, alkyloxy containing 1 to 10 carbon atoms, acyloxy containing 1 to 10 carbon atoms, and alkylthio containing 1 to 10 carbon atoms, among others. The preferred are halogen, nitro, N-protected amino, C₁₋₁₀ alkyl, and C₁₋₁₀ alkyloxy. The number of substituents that may be present is 0 to 3.

The C₁₋₁₀ alkyl moieties of the above-mentioned C₁₋₁₀ alkylamino, C₁₋₁₀ alkyl, C₁₋₁₀ alkyloxy, and C₁₋₁₀ alkylthio groups may be the same C₁₋₁₀ alkyl groups as those mentioned for R. Referring to said dialkylamino containing 2 to 10 carbon atoms, the two alkyl groups are selected so that the total number of carbon atoms will be 2 to 10. The acyloxy containing 1 to 10 carbon atoms includes formyloxy, acetyloxy, propionyloxy, butyryloxy, valeryloxy, pivaloyloxy, and hexanoyloxy, among others.

The protective group of the N-protected amino group includes the protective groups mentioned in Theodora W. Green: Protective Groups in Organic Synthesis, 2nd Ed., JOHN WILEY & SONS, 1990, 309-384. Specifically, there may be mentioned aralkyl type protective groups such as benzyl, phenethyl, triphenylmethyl, etc.; sulfonyl type protective groups such as methanesulfonyl, trifluoromethanesulfonyl, benzenesulfonyl, p-toluenesulfonyl, o-nitrobenzenesulfonyl, m-nitrobenzenesulfonyl, p-nitrobenzenesulfonyl, etc.; carbamate type protective groups such as methoxycarbonyl, ethoxycarbonyl, tert-butoxycarbonyl, benzyloxycarbonyl, etc.; and acetyl type protective groups such as phthaloyl, acetyl, chloroacetyl, trifluoroacetyl, pivaloyl, benzoyl, and so forth.

Referring to the above formulas (3a) and (4a), Py represents a pyridyl group which may be substituted, namely 2-pyridyl group which may be substituted, 3-pyridyl group which may be substituted, and 4-pyridyl group which may be substituted. The preferred is 2-pyridyl group. The substituent or substituents that may be present on the Py nucleus include the same substituent groups as those mentioned above for Ar.

Furthermore, in the above formulas (1), (2), (4), (4a), and (5), each * represents an asymmetric carbon atom.

The optically active N-pyridyl lactamide derivative of the general formula (4a) is a novel compound which had not been described in the literature but was found by the present inventors to be of value to the production of the optically active N-pyridyl-1-amino-2-propanol derivative of the general formula (5a) : 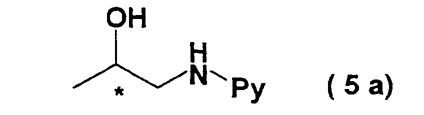 (in the formula, Py represents 2-pyridyl group which may be substituted, 3-pyridyl group which may be substituted, or 4-pyridyl group which may be substituted; * represents an asymmetric carbon atom).

Now, the enantiomer content of the compound represented by the above formula (4a) is usually not more than 10%, preferably not more than 3%, more preferably not more than 1%. Moreover, the preferred absolute configuration of the compound represented by the above formula (4a) or (5a) is (S).

Furthermore, the optically active N-pyridyllactamide derivative (4a) is preferably isolated as crystals.

The process for producing an optically active N-aryl-1-amino-2-propanol derivative according to the invention is now described.

The process for producing an optically active N-aryllactamide derivative is now described in the first place.

The optically active N-aryllactamide derivative (4) may be produced by reacting an optically active lactate derivative of the above formula (1) or an optically active lactic acid acetal derivative of the above formula (2) with an arylamine derivative represented by the above formula (3).

As the optically active lactate derivative (1), a commercial product may be used as such or a commercial optically active lactic acid may be esterified according to the ordinary process and used.

The optically active lactic acid acetal derivative (2) can be easily synthesized by, for example, reacting the optically active lactic acid, which is inexpensive and readily available, with 2,2-dimethoxypropane under heating (J. Org. Chem., 1990, 55, 5871).

The molar ratio of the optically active lactate derivative (1) or optically active lactic acid acetal derivative (2) to the arylamine derivative (3) for use in the invention is preferably 1:5 to 5:1, more preferably 1:1 to 3:1, still more preferably 1.5:1 to 2.5:1.

Between the optically active lactate derivative (1) and the optically active lactic acid acetal derivative (2), it is more advantageous to use the optically active lactate derivative (1).

The reaction solvent which can be used for this reaction includes alcohols such as methanol, ethanol, isopropyl alcohol, n-butanol, etc.; hydrocarbons such as hexane, heptane, benzene, toluene, etc.; ethers such as diethyl ether, diisopropyl ether, di-n-butyl ether, dimethoxyethane, diethylene glycol dimethyl ether, tetrahydrofuran, 1,4-dioxane, methyl tert-butyl ether, etc.; halogen-containing solvents such as methylene chloride, chloroform, 1,1,1-trichloroethane, carbon tetrachloride, 1,2-dichloroethane, monochlorobenzene, etc.; esters such as formic esters, e.g. ethyl formate etc., acetic esters, e.g. methyl acetate, ethyl acetate, n-propyl acetate, isopropyl acetate, n-butyl acetate, tert-butyl acetate, etc., propionic esters, e.g. methyl propionate, ethyl propionate, n-propyl propionate, isopropyl propionate, n-butyl propionate, isobutyl propionate, etc., γ-butyrolactone, etc.; ketones such as acetone, methyl ethyl ketone, diethyl ketone, cyclopentanone, cyclohexanone, dibutyl ketone, etc.; nitrogen-containing solvents such as dimethylformamide, acetamide, formamide, acetonitrile, propionitrile, etc.; aprotic polar solvents such as dimethyl sulfoxide, N-methylpyrrolidone, hexamethylphosphoric triamide, etc.; and amines represented by triethylamine, among others. The above-mentioned reaction solvents may be used each independently or in a combination of two or more different species.

In this connection, the reaction under heating of the arylamine derivative (3) with the optically active lactate derivative (1) or the optically active lactic acid acetal derivative (2) gives rise to the byproduct enantiomer so that the optical purity of the product optically active N-aryllactamide derivative (4) tends to be sacrificed. This tendency becomes more and more pronounced as the reaction temperature is increased. On the other hand, use of a low reaction temperature alleviates the depression of optical purity but tends to reduce the reaction velocity.

The intensive investigation by the inventors of the present invention revealed that while a reaction solvent is generally used for dissolving the starting material and reaction product and/or promoting the reaction, the amount of the reaction solvent is preferably as small as possible in order that the reaction may be successfully carried through and that it is particularly advantageous to conduct the reaction in the absence of a reaction solvent. It was also found that the reaction is preferably conducted within a given temperature range. Accordingly the inventors established a very efficient production protocol by which the reaction can be conducted at a consistently high rate and in high concentration while the formation of the byproduct enantiomer is well suppressed.

The level of use of said reaction solvent is generally not over 10 times weight, preferably not over 5 times weight, more preferably not over twice weight, still more preferably not over equal weight, especially preferably not over half weight, particularly not over 0.2 times weight, of the arylamine derivative (3). Of course, it is most advantageous to conduct the reaction in the absence of a reaction solvent.

The reaction temperature for this reaction is preferably 70 to 130°C, more preferably 80 to 120°C, still more preferably 90 to 110°C, especially preferably 95 to 105°C.

The reaction time for this reaction is preferably 1 hour to 10 days, more preferably 1 hour to 5 days, still more preferably 1 hour to 3 days.

The optically active N-aryllactmide derivative (4) obtained by the above reaction may be purified according to the ordinary process.

Then, the process for purifying the optically active N-aryllactamide derivative (4), particularly said optically active N-pyridyllactamide derivative (4a), in accordance with the invention is described below.

The optically active N-pyridyllactamide derivative (4a) produced by reacting said optically active lactate derivative of the formula (1) or optically active lactic acid acetal derivative of the formula (2) with said aminopyridine derivative of the formula (3a) tends to contain various impurities derived from side reactions and decomposition in the course of production. Particularly under the above-mentioned reaction conditions, said compound (4a) tends to undergo racemization and, therefore, contain its enantiomer. Furthermore, the reaction mixture containing said compound (4a) usually contains not only this objective product compound but also the unreacted starting compounds [optically active lactate derivative (1) or optically active lactic acid acetal derivative (2) and aminopyridine derivative (3a)] and byproducts. Removal of structurally analogous impurity (analogous compounds) is generally difficult and in order that such impurity may be removed to isolate the objective compound of high quality, an effective isolation and purification procedure is required.

The inventors of the present invention explored in earnest for such a procedure and ultimately developed the following protocol comprising a washing procedure, an extraction procedure and a crystallization procedure, in accordance with which various impurities such as unreacted starting compounds, byproducts, and enantiomer can be efficiently removed from the reaction mixture containing the objective compound (4a) to give the compound (4a) of high quality with good efficiency. The washing procedure, extraction procedure, and crystallization procedure are now explained in that order.

The washing procedure is described in the first place.

The preferred process for removing the unreacted starting material optically active lactate derivative (1) or optically active lactic acid acetal derivative (2) and byproducts derived from the starting material comprises bringing the reaction product mixture under an acidic condition in a biphasic system comprising water and an organic solvent and removing the organic solvent layer by fractionating the optically active N-pyridyllactamide derivative (4a) retained in the aqueous layer. Thus, for example, water and an organic solvent are added to the reaction mixture, the system is then acidified with an acid and allowed to be separated, and the organic solvent layer is removed.

The acid which may be used for acidification is not particularly restricted but includes, *inter* alia, various inorganic acids such as hydrochloric acid, sulfuric acid, phosphoric acid, etc. and organic acids such as formic acid, acetic acid, trifluoroacetic acid, p-toluenesulfonic acid, methanesulfonic acid, and so forth. Among these acids, inorganic acids are preferred and hydrochloric acid is particularly preferred among them.

The acidic conditions mentioned above may vary somewhat depending upon the kinds and amounts of concomitant impurities but, among other factors, generally are not over pH 2, preferably not over pH 1.

The organic solvent referred to above includes hydrocarbons such as hexane, heptane, benzene, toluene, etc.; ethers such as diethyl ether, diisopropyl ether, di-n-butyl ether, dimethoxyethane, diethylene glycol dimethyl ether, tetrahydrofuran, 1,4-dioxane, methyl tert-butyl ether, etc.; halogen-containing solvents such as methylene chloride, chloroform, 1,1,1-trichloroethane, carbon tetrachloride, 1,2-dichloroethane, monochlorobenzene, etc.; esters, e.g. formic esters such as ethyl formate etc., acetic esters such as methyl acetate, ethyl acetate, n-propyl acetate, isopropyl acetate, n-butyl acetate, tert-butyl acetate, etc., and propionic esters such as methyl propionate, ethyl propionate, n-propyl propionate, isopropyl propionate, n-butyl propionate, isobutyl propionate, etc., γ-butyrolactone, etc., and ketones such as acetone, methyl ethyl ketone, diethyl ketone, cyclopentanone, cyclohexanone, dibutyl ketone, and so forth. The preferred are ethers, halogen-containing solvents, and esters. Among these, esters are particularly preferred. As such esters, any of formic esters, acetic esters, and propionic esters may be used but acetic esters are particularly preferred, and ethyl acetate is the most preferred. These organic solvents may be used each independently or in a combination of two or more species.

By the above procedure, the unreacted starting material optically active lactate derivative (1) or optically active lactic acid acetal derivative (2) and the byproducts derived therefrom may be removed substantially without incurring loss of the objective compound (4a). It should be noticed that this procedure is important for achieving a successful crystallization in the crystallization procedure described hereinafter and if this procedure is not performed, the crystallization tends to become difficult.

The extraction procedure is now described.

To remove the unreacted starting material aminopyridine derivative (3a) which cannot be removed by the above washing procedure, the following extraction procedure is preferably carried out. The specific procedure comprises bringing the optically active N-pyridyllactamide derivative (4a) containing the aminopyridine derivative (3a) under a weakly acidic condition in a biphasic system comprising water and an organic solvent (for example, a system established by adding an organic solvent to the aqueous layer available after said washing procedure) to extract the optically active N-pyridyllactamide derivative (4a) into the organic solvent layer while the aminopyridine derivative (3a) is retained in the aqueous layer.

The weakly acidic conditions mentioned above may vary somewhat depending on the kinds and amounts of impurities, among other factors, but are generally pH 3 to 6, preferably pH 3 to 5.

The base for use in adjusting the system to the weakly acidic conditions is not particularly restricted but includes alkali metal hydroxides such as sodium hydroxide, potassium hydroxide, etc.; alkali metal carbonates such as sodium carbonate, potassium carbonate, etc.; and alkali metal hydrogencarbonates such as sodium hydrogencarbonate etc., among others.

The organic solvent for use in the extraction procedure includes hydrocarbons such as hexane, heptane, benzene, toluene, etc.; ethers such as diethyl ether, diisopropyl ether, di-n-butyl ether, dimethoxyethane, diethylene glycol dimethyl ether, tetrahydrofuran, 1,4-dioxane, methyl tert-butyl ether, etc.; halogen-containing solvents such as methylene chloride, chloroform, 1,1,1-trichloroethane, carbon tetrachloride, 1,2-dichloroethane, monochlorobenzene, etc.; esters, e.g. formic esters such as ethyl formate etc., acetic esters such as methyl acetate, ethyl acetate, n-propyl acetate, isopropyl acetate, n-butyl acetate, tert-butyl acetate, etc., and propionic esters such as methyl propionate, ethyl propionate, n-propyl propionate, isopropyl propionate, n-butyl propionate, isobutyl propionate, etc., γ-butyrolactone, etc.; and ketones such as acetone, methyl ethyl ketone, diethyl ketone, cyclopentanone, cyclohexanone, dibutyl ketone, and so forth. The preferred are ethers, halogen-containing solvents, and esters. Among these, esters are particularly preferred. As such esters, any of formic esters, acetic esters, and propionic esters may be used but acetic esters are particularly preferred, ethyl acetate is the most preferred. These organic solvents can be used each independently or in a combination of two or more species.

By removing the reaction solvent and extraction solvent from the resulting extract, for example by concentration under reduced pressure, the objective compound of high chemical purity may be recovered with good efficiency.

To further improve the quality, that is to say chemical purity and optical purity, of the optically active N-pyridyllactamide derivative (4a) recovered by the above isolation-purification process (washing and extraction), a crystallization procedure is preferably carried out. This crystallization procedure is now described.

Crystallization of the optically active N-pyridyllactamide derivative (4a) is not necessarily quite easy but the inventors of the present invention found surprisingly that the objective compound may be recovered very advantageously as a crystal crop by carrying out the above-described isolation-purification process, especially the washing procedure.

In addition, by causing the optically active N-pyridyllactamide derivative (4a) containing at least its impurity enantiomer to crystallize by using an organic solvent in accordance with the invention, it is possible to remove the impurity inclusive of said enantiomer and recover the optically active N-pyridyllactamide derivative (4a) of high purity as crystals.

The organic solvent (crystallization solvent) which can be used in this crystallization procedure is not particularly restricted but includes, inter alia, aromatic hydrocarbons such as benzene, toluene, o-xylene, m-xylene, p-xylene, ethylbenzene, cumene, n-butylbenzene, 1,3,5-mesitylene, etc.; alcohols such as methanol, ethanol, isopropyl alcohol, n-butanol, etc.; ethers such as diethyl ether, diisopropyl ether, di-n-butyl ether, dimethoxyethane, diethylene glycol dimethyl ether, tetrahydrofuran, 1,4-dioxane, methyl tert-butyl ether, etc.; halogen-containing solvents such as methylene chloride, chloroform, carbon tetrachloride, 1,2-dichloroethane, 1,1,1-trichloroethane, monochlorobenzene, etc.; esters, e.g. formic esters such as ethyl formate etc., acetic esters such as methyl acetate, ethyl acetate, n-propyl acetate, isopropyl acetate, n-butyl acetate, tert-butyl acetate, etc., and propionic esters such as methyl propionate, ethyl propionate, n-propyl propionate, isopropyl propionate, n-butyl propionate, isobutyl propionate, etc., γ-butyrolactone, etc.; ketones such as acetone, methyl ethyl ketone, diethyl ketone, cyclopentanone, cyclohexanone, dibutyl ketone, etc.; nitrogen-containing solvents such as dimethylformamide, acetamide, formamide, acetonitrile, propionitrile, etc.; and aprotic polar solvents such as dimethyl sulfoxide, N-methylpyrrolidone, hexamethylphosphoric triamide, and so forth. These crystallization solvents may be used each independently or in a combination of two or more species.

Meanwhile, it is known that because of its poor crystallizability, said compound (4a) tends to cause various troubles: it is made oily in the case of crystallization and, even if it can be crystallized, gives a viscous slurry leading to a failure to obtain the expected purification effect, it takes a long time for crystals to separate out, and the obtained crystal cannot be neatly dried. To overcome these disadvantages, it is preferable to use, as a solvent for crystallization, an aromatic hydrocarbon such as benzene, toluene, o-xylene, m-xylene, p-xylene, ethylbenzene, cumene, n-butylbenzene, 1, 3, 5-mesitylene, or the like, more preferably an aromatic hydrocarbon of 6 to 10 carbon atoms. From global points of view taking solvent cost, ease of handling, etc. into consideration, it is still more preferable to use toluene. Of course, these solvents may be used each independently or in a combination of two or more species.

Particularly when the above-mentioned aromatic hydrocarbon is used, the high-degree purification of said compound (4a), namely effective removal of various impurity substances, especially the enantiomer of said compound (4a), can be neatly accomplished.

The level of use of the above crystallization solvent is not particularly restricted but need only be high enough to insure adequate fluidity of the system at completion of crystallization of said compound (4a). This requirement, coupled with the economic consideration, suggests that it is preferable to use the solvent 1 to 20 times weight, more preferably about 2 to 10 times weight, of said compound (4a) prior to crystallization.

When the crystallization is carried out using said aromatic hydrocarbon, an auxiliary solvent may be used for the purpose of improving at least one of yield, treating concentration, slurry fluidity in the crystallization, of the compound (4a), and purity and physical properties of product crystals. This auxiliary solvent may be added as needed to the crystallization slurry or in admixture with said crystallization solvent before the start of crystallization.

This auxiliary solvent is not particularly restricted but is preferably selected from among aliphatic hydrocarbons such as pentane, petroleum ether, neopentane, hexane, cyclohexane, methylcyclohexane, heptane, cycloheptane, octane, isooctane, nonane, decane, etc.; esters, e.g. formic esters such as ethyl formate etc., acetic esters such as methyl acetate, ethyl acetate, n-propyl acetate, isopropyl acetate, n-butyl acetate, tert-butyl acetate, etc., and propionic esters such as methyl propionate, ethyl propionate, n-propyl propionate, isopropyl propionate, n-butyl propionate, isobutyl propionate, etc., γ-butyrolactone, etc.; and amines such as ethylamine, isopropylamine, diethylamine, triethylamine, aniline, pyridine, and so forth.

The use of an aliphatic hydrocarbon and/or an ester is advantageous for improving the yield and treating concentration of said compound (4a), and the use of an amine is advantageous for improving the purity, particularly optical purity, of the obtained crystal.

The aliphatic hydrocarbon mentioned above is preferably one containing 5 to 10 carbon atoms, more preferably 6 to 8 carbon atoms. As the ester, any of formic, acetic, and propionic esters may be used with advantage but acetic esters are particularly preferred and the use of ethyl acetate is the most advantageous. As the amine mentioned above, triethylamine, isopropylamine, diethylamine and pyridine, and so forth, are preferred. These may be used each independently or in a combination of two or more species.

The proper level of use of said auxiliary solvent may be easily established by simple experimentation. From yield and slurry fluidity points of view, the level of use of said auxiliary solvent in terms of its ratio to the crystallization solvent by weight ((weight of auxiliary solvent)/(weight of crystallization solvent)) is generally not more than 1, preferably not more than 0.5, still more preferably 0.01 to 0.2, as of the time of completion of the procedure for crystallization of said compound (4a).

The crystallization according to the invention may be achieved by the ordinary crystallization technology, such as cooling crystallization, concentrating crystallization, and neutralizing crystallization, or a combination of such techniques. In. this connection, the above-mentioned crystallization by concentration may be crystallization by a process in which solution consisting of a solvent other than the above-mentioned crystallization solvent is transferred to solution comprising the above-mentioned crystallization solvent. Among the above-mentioned crystallization techniques, it is particularly recommendable to perform crystallization by cooling either alone or in combination with crystallization by some other technique. In this crystallization procedure, seed crystals may be added.

The crystallization procedure according to the invention can be conducted around room temperature. Where necessary, heating or cooling may be carried out and the crystallization procedure can be carried out with advantage within the range of, for example, -30°C to 100°C, preferably -20°C to 80°C.

Furthermore, the crystallization is preferably carried out under intense agitation. The intensity of stirring or agitation is not particularly restricted but the stirring may be made usually at not less than 0.05 kW/m³, preferably at not less than 0.2 kW/m³.

The crystallization speed is not particularly restricted but in order that crystals of high quality may be recovered, it is preferably controlled so that the crystallization yield per hour will not more than 50% of the total obtained crystal, preferably not more than 25%.

The compound (4a) thus obtained may be isolated by liquid-solid separation and, where necessary, the cake may be washed and dried. The procedure for said liquid-solid separation is not particularly restricted but includes pressure filtration, suction filtration, and centrifugation, among other techniques. The procedure for said drying is preferably drying under reduced pressure (vacuum drying) at a temperature not more than about 60°C.

The technology of producing an optically active N-aryl-1-amino-2-propanol derivative (5) according to the present invention is now described.

The optically active N-aryl-l-amino-2-propanol derivative (5) can be produced by treating the optically active N-aryllactamide derivative (4), as prepared by the process described above, with a reducing agent.

The reducing agent mentioned just above may for example be a borane derivative, an aluminum hydride complex compound, or a boron hydride complex compound, among others. The preferred is a borane derivative or an aluminum hydride complex compound and the more preferred is a borane derivative.

The borane derivative includes borane, borane-THF complex, borane-dimethyl sulfide, thexylborane, disiamylborane, 9-borabicyclo[3.3.1]nonane, catecholborane, and (di)isopinocamphenylborane. The preferred are borane, borane-THF complex, and borane-dimethyl sulfide and the more preferred are borane and borane-THF complex. The "borane" in these borane derivatives includes the dimer and polymer, for example diborane.

The aluminum hydride complex compound includes lithium aluminum hydride, lithium trimethoxyaluminum hydride, lithium triethoxyaluminum hydride, lithium tri-t-butoxyaluminum hydride, and sodium bis(2-methoxyethoxy)aluminum hydride (™Red-Al, Vitride). Preferred is lithium aluminum hydride.

The boron hydride complex compound includes sodium borohydride, potassium borohydride, sodium trimethoxyboron hydride, sodium borohydride cyanide, and sodium triacetoxyboron hydride. The preferred are sodium borohydride and potassium borohydride.

The level of use of said reducing agent varies with different reducing agents but from economic points of view, it is not more than 10 equivalents relative to the optically active N-aryllactamide derivative (4). It is more preferably not more than 5 equivalents, still more preferably 1 to 3 equivalents.

The borane derivative mentioned above has a high reducing power as a reducing agent but, the other way round, is so sensitive to air and water that it is hard to store and commercially tends to cause troubles in handling.

Therefore, in the industrial application of the above borane derivative, it is recommendable to generate the borane derivative by reacting a boron hydride complex compound with an acid.

The acid mentioned just above is not particularly restricted but includes inorganic acids, such as hydrochloric acid, sulfuric acid, phosphoric acid, etc., organic acids, such as methanesulfonic acid, benzenesulfonic acid, p-toluenesulfonic acid, acetic acid, trifluoroacetic acid, etc.; Lewis acids, such as BF₃ · (EtO)₂, cobalt chloride, titanium chloride, etc.; iodine and iodine compounds such as methyl iodide, and so forth. The preferred are inorganic acids and organic acids (preferably sulfonic acids), the still more preferred are sulfuric acid and methanesulfonic acid, and the particularly preferred is sulfuric acid.

The boron hydride complex compound which may be used for generating the borane derivative includes sodium borohydride, potassium borohydride, sodium trimethoxyborohydride, sodium cyanoborohydride, sodium triacetoxyborohydride, and so forth. The preferred are sodium borohydride and potassium borohydride.

Referring to the combination of a boron hydride complex compound with an acid, the combination of sodium borohydride with sulfuric acid or that of sodium borohydride with methanesulfonic acid is preferred and the combination of sodium borohydride with sulfuric acid is the most preferred.

The ratio of the boron hydride complex compound to the acid (boron hydride complex compound:acid) depends on the specific combination but is generally 1:1 to 10:1, preferably 1:1 to 5:1, more preferably 1:1 to 2:1.

From economic points of view, the level of use of the boron hydride complex compound relative to the optically active N-aryllactamide derivative (4) is preferably not over 10 equivalents, more preferably not over 5 equivalents, still more preferably 1 to 3 equivalents.

The reaction temperature for the above reduction reaction is preferably -30°C to 150°C, more preferably -20°C to 100°C, still more preferably -10°C to 80°C. The reaction time is preferably 1 to 72 hours, more preferably 1 to 24 hours.

The reaction solvent which can be used in conducting this reduction reaction includes water; alcohols such as methanol, ethanol, isopropyl alcohol, n-butanol, etc.; hydrocarbons such as hexane, heptane, benzene, toluene, etc.; ethers such as diethyl ether, diisopropyl ether, di-n-butyl ether, dimethoxyethane, diethylene glycol dimethyl ether, tetrahydrofuran, 1,4-dioxane, methyl tert-butyl ether, etc.; halogen-containing solvents such as methylene chloride, chloroform, 1,1,1-trichloroetane, carbon tetrachloride, 1,2-dichloroethane, monochlorobenzene, etc.; esters, e.g. formic esters such as ethyl formate etc., acetic esters such as methyl acetate, ethyl acetate, n-propyl acetate, isopropyl acetate, n-butyl acetate, tert-butyl acetate, etc., and propionic esters such as methyl propionate, ethyl propionate, n-propyl propionate, isopropyl propionate, n-butyl propionate, isobutyl propionate, etc., γ-butyrolactone, etc.; and ketones such as acetone, methyl ethyl ketone, diethyl ketone, cyclopentanone, cyclohexanone, dibutyl ketone, and so forth. The preferred are ethers and the more preferred are cyclic ethers such as tetrahydrofuran and 1, 4-dioxane. The still more preferred is tetrahydrofuran. These reaction solvents may be used each independently or two or more of them may be used in combination.

The level of use of the reaction solvent is not particularly restricted but preferably not over 100 times weight, more preferably not over 50 times weight, still more preferably 1 to 20 times weight, of the optically active N-aryllactamide derivative (4).

The efficient process for acquiring the optically active N-aryl-1-amino-2-propanol derivative (5) resulting from the above reaction is now described.

When a boron hydride complex compound or a borane derivative is used as the reducing agent in the reduction of said compound (4) to said compound (5), it may happen that the product optically active N-aryl-1-amino-2-propanol derivative (5) coordinates with boron to lower the yield (extraction yield, distillation yield) of the product. Particularly the boron component derived from the reducing agent which may occur in a slight amount in the extract tends to lower the distillation yield of the optically active N-aryl-1-amino-2-propanol derivative (5).

To overcome this drawback of yield reduction and acquire the optically active N-aryl-1-amino-2-propanol derivative (5) of high quality with improved efficiency, it is advisable to carry out the following extraction procedure and distillation procedure. In this connection, it is especially effective to carry out an acid treatment to be described below in conjunction with said extraction procedure and a treatment with water and/or an alcohol to be described below in conjunction with said distillation procedure.

The extraction procedure and the acid treatment to be carried out as an adjunct are now described in the first place.

In order to remove the coordinating boron from a boron-coordinated complex of the optically active N-aryl-1-amino-propanol derivative (5) occurring in the reaction mixture and thereby acquire the optically active N-aryl-1-amino-propanol derivative (5), this complex compound is preferably treated under an acidic condition in carrying out the extraction procedure. For example, it is good practice to have an acid to be brought into contact with said complex compound in a solvent, preferably a biphasic system comprising water and an organic solvent, and stir the mixture.

The acid to be brought into contact is not particularly restricted but includes inorganic acids, such as hydrochloric acid, sulfuric acid, phosphoric acid, etc., and organic acids, such as formic acid, acetic acid, trifluoroacetic acid, p-toluenesulfonic acid, methanesulfonic acid and so forth. Among these acids, an inorganic acid, especially hydrochloric acid, is preferred.

The acidic conditions mentioned above, in terms of pH, are preferably not over pH 3, more preferably not over pH 2, still more preferably not over pH 1.

The temperature for said acid treatment is not particularly restricted but the higher the temperature is, the shorter is the time in which the acid treatment can be carried through. The acid treatment can be carried out at a temperature of preferably not below 0°C, more preferably 5 to 70°C, still more preferably 20 to 60°C.

The solvent for use in the above acid treatment includes the same solvents as those mentioned for said reduction reaction.

After the above acid treatment, the optically active N-aryl-1-amino-2-propanol derivative (5) can be extracted with good efficiency by the following extraction procedure.

The extraction procedure using a biphasic system comprising water and an organic solvent, for instance, comprises adding a base to the system to establish weakly acidic to basic conditions and subjecting the system to phase separation retaining the inorganic salt and other impurity in the aqueous layer and recovering the organic solvent layer containing the optically active N-aryl-1-amino-2-propanol derivative (5).

The base which can be used for this purpose may be invariably an inorganic base and an organic base but is preferably an inorganic base. As specific examples, alkali metal hydroxides such as sodium hydroxide, potassium hydroxide, etc.; alkali metal carbonates such as sodium carbonate, potassium carbonates; and alkali metal hydrogencarbonates, such as sodium hydrogencarbonate etc. may be mentioned. Among these, alkali metal hydroxides are preferred and sodium hydroxide is particularly preferred.

The weakly acidic to basic conditions mentioned above mean the range of pH 5 to pH 13, preferably pH 6 to 11, more preferably pH 7 to 10, still more preferably pH 8 to 9.

The organic solvent referred to above includes hydrocarbons such as hexane, heptane, benzene, toluene, etc.; ethers such as diethyl ether, diisopropyl ether, di-n-butyl ether, dimethoxyethane, diethylene glycol dimethyl ether, tetrahydrofuran, 1,4-dioxane, methyl tert-butyl ether, etc.; halogen-containing solvents such as methylene chloride, chloroform, 1,1,1-trichloroethane, carbon tetrachloride, 1,2-dichloroethane, monochlorobenzene, etc.; esters, e.g. formic esters such as ethyl formate etc., acetic esters such as methyl acetate, ethyl acetate, n-propyl acetate, isopropyl acetate, n-butyl acetate, tert-butyl acetate, etc., and propionic esters such as methyl propionate, ethyl propionate, n-propyl propionate, isopropyl propionate, n-butyl propionate, isobutyl propionate, etc., γ-butyrolactone, etc.; and ketones such as acetone, methyl ethyl ketone, diethyl ketone, cyclopentanone, cyclohexanone, dibutyl ketone, and so forth. The preferred are ethers, halogen-containing solvents, and esters and the more preferred are esters. With regard to the esters, any of formic, acetic, and propionic esters can be used with advantage, although acetic esters are particularly advantageous and ethyl acetate is the most useful.

Now, the distillation procedure and the associated treatment with water and/or alcohol are described.

Distillation of the optically active N-aryl-1-amino-2-propanol derivative (5) involves the problem that the contaminant boron component lowers distillation yield. Therefore, in order that the distillation yield may be maximized, the concomitant (residual) boron component is preferably lowered or removed. In conducting the distillation, the boron component content is brought down preferably to a level not over 10 mole %, more preferably not more than 5 mole %, relative to the optically active N-aryl-1-amino-2-propanol derivative (5).

With regard to the process for lowering or removing the boron component, it is advantageous to treat an extract containing the optically active N-aryl-1-amino-2-propanol derivative (5) (preferably the extract obtained by said extraction procedure) or a concentrate thereof with water and/or an alcohol.

The above treatment with water can be effected by, for example, washing said extract containing the optically active N-aryl-1-amino-2-propanol derivative (5) or said concentrate with an appropriate amount of water. In this case, said boron component is removed into the aqueous layer.

The above treatment with water is carried out advantageously under a weakly acidic to basic condition; generally at pH 5 to 13, preferably at pH 6 to 11, more preferably at pH 7 to 10.

The quantity of water to be used is not particularly restricted but may, for example, be about 0.01 to 1 times weight, preferably 0.01 to 0.2 times weight, of said extract or concentrate. The temperature for the treatment with water is not particularly restricted but may be somewhere between the solidification point and boiling point of the system, preferably 0 to 50°C.

The treatment with an alcohol mentioned above can be effected by, for example, adding an appropriate amount of an alcohol to an extract containing the optically active N-aryl-1-amino-2-propanol derivative (5) or a concentrate thereof and concentrating the mixture. In this case, said boron component reacts with the alcohol to become a low-boiling compound and is evaporated off as such.

The alcohol for use is not particularly restricted but monohydric alcohols of 1 to 3 carbon atoms, such as methanol, ethanol, n-propyl alcohol, and isopropyl alcohol, are preferred.

The level of use of the alcohol cannot be stated in general terms because it depends on the kind of alcohol, the kinds and amounts of contaminant substances derived from the reducing agent, and other factors but it is generally preferable to use at least an equimolar amount relative to the amount of said contaminant substances derived from the reducing agent. It may be used in excess over said contaminant substances. The level of use of said alcohol relative to the optically active N-aryl-1-amino-2-propanol derivative (5) may for example be at least equal to the weight, preferably not less than 3 times weight, of the derivative (5).

By performing said treatment with water and/or alcohol in the above manner, the boron component can be lowered or removed to the above-mentioned preferred range.

Lastly, the distillation procedure (inclusive of fractional distillation procedure) is described below.

The optically active N-aryl-1-amino-2-propanol derivative (5) obtained by the above reduction reaction contains the reaction byproduct arylamine derivative (3). For example, the optically active N-pyridyl-1-amino-2-propanol derivative (5a) contains the aminopyridine derivative (3a).

In the preliminary investigation carried out for the removal of said contaminant by the distillation procedure, the inventors of the present invention encountered the problem that the efficiency of removal is very low. Accordingly the inventors intensively investigated the above problem and found that the problem is caused by the following mechanism. Thus, in the distillation procedure, the comparatively low-boiling arylamine derivative (3) (for example the aminopyridine derivative (3a), particularly 2-aminopyridine) is first distilled (first drop) but since its melting point is comparatively high, it is partially solidified in the distillate line (inclusive of the condenser) of the still and finds its way into the objective fraction (product) in the subsequent recovery (recovery of objective fraction) of the optically active N-aryl-1-amino-2-propanol derivative (5) (for example, the optically active N-pyridyl-1-amino-2-propanol derivative (5a), particularly optically active N-(2-pyridyl)-1-amino-2-prpanol, to cause the above contamination problem.

Therefore, in order that the objective compound of high quality may be acquired in this distillation procedure, it is good practice to cause said arylamine derivative (3) (for example the aminopyridine derivative (3a), particularly 2-aminopyridine) to be distilled and recovered in the first place and, then, through the distillate line (inclusive of the condenser) essentially not contaminated by the arylamine derivative (3), cause the optically active N-aryl-1-amino-2-propanol derivative (5) (for example the optically active N-pyridyl-1-amino-2-propanol derivative (5a), particularly the optically active N-(2-pyridyl)-1-amino-2-propanol), to be distilled and recovered. Specifically, the following two alternative processes, among others, may be mentioned.
1. The above-mentioned arylamine derivative (3) and optically active N-aryl-1-amino-2-propanol derivative (5) are distilled and recovered from independent distillate lines (inclusive of condensers) or when both are to be distilled from one and the same distillate line (inclusive of the condenser), the distillate line (inclusive of the condenser) is washed following distillation of the arylamine derivative (3) and the optically active N-aryl-1-amino-2-propanol derivative (5) is then distilled and recovered. The preferred is the mode in which the two compounds are distilled and recovered via independent distillate lines (inclusive of condensers).
2. With the temperature of the distillate line (inclusive of the condenser) of the still being maintained at a level not lower than the melting point of the arylamine derivative (3) (for example the aminopyridine derivative (3a), particularly 2-aminopyridine) or the distillate composed predominantly thereof, the optically active N-aryl-1-amino-2-propanol derivative (5) is distilled. In this connection, for example said arylamine derivative (3) is 2-aminopyridine or 3-aminopyridine, the above-mentioned temperature level is not below about 55°C, preferably not below about 60°C.

Of the above two processes, the first process 1 is preferred.

Of course, these distillation processes may be used in a suitable combination.

Although it depends on the quality prior to distillation and the expected quality of the product obtainable by distillation, the optically active N-aryl-1-amino-2-propanol derivative (5) of high quality can thus be obtained with the percent loss of the derivative (5) upon the first drop off being kept at generally not more than 5%, preferably not more than 3%.

From the standpoint of thermal stability, the distillation (inclusive of fractional distillation) of the optically active N-aryl-1-amino-2-propanol derivative (5) is preferably conducted under reduced pressure.

### BEST MODE FOR CARRYING OUT THE INVENTION

The following examples illustrate the present invention in further detail without defining the scope of the invention.

In the following examples, analyses were made by liquid chromatography (HPLC). The conditions and parameter settings for quantitative analyses (percent conversion, yield, content, etc.) were: column = Develosil ODS-HG-3 4.6 mm ϕ x 150 mm (product of Nomura Chemical Co.), eluent = 0.1% aq. sol. of potassium dihydrogen phosphate/acetonitrile = 7/3, flow rate = 0. 5 ml/min, temperature = 40°C, detection = UV 210 nm.

The conditions and parameter settings for optical purity analyses were: column = Chiralpak AD 4.6 mm ϕ x 250 mm (product of Daicel Chemical Industries, Ltd.), eluent: hexane/isopropyl alcohol = 90/10, flow rate = 0.5 ml/min, temperature = 40°C, detection = UV 210 nm.

### Example 1 Production of (S)-N-(2-pyridyl)lactamide

(S)-Methyl lactate (optical purity = 98.9% ee) 10.4 g (100 mmol) and 4.71 g (50 mmol) of 2-aminopyridine were admixed and reacted under heating at 90°C for 3 days. Analysis of the reaction mixture by HPLC at completion of the reaction revealed that the conversion rate was 74.9%.

This reaction mixture was dissolved in 100 ml of 1N-hydrochloric acid and the residual (S)-methyl lactate was removed by extraction with 100 ml of ethyl acetate (pH ≤ 1). The aqueous layer was adjusted to pH 4.5 with aqueous solution of NaOH and extracted twice with 100 ml each of ethyl acetate (2-aminopyridine was retained in the aqueous layer). The organic layer was concentrated under reduced pressure to give 6.1 g of pale yellow solid.

A portion of the above product was purified by silica gel chromatography to give white crystals (30 mg) of (S)-N-(2-pyridyl)lactamide. The optical purity as analyzed by HPLC was 93.8% ee.
¹H-NMR (CDCl₃, 400 MHz/ppm): 1.57 (3H, d), 4.44 (1H, q), 5.39 (1H, bs), 7.09 (1H, dd), 7.75 (1H, dd), 8.25 (1H, d), 8.30 (1H, d), 9.48 (1H, bs). mp = 102°C to 104°C

### Example 2 Production of (S)-N-(2-pyridyl)lactamide

(S)-Methyl lactate (optical purity = 98.9% ee) 31.5 g (302 mmol) and 14.2 g (151 mmol) of 2-aminopyridine were admixed and reacted under heating at 80°C for 5 days. Analysis of the reaction mixture at completion of the reaction by HPLC revealed that the yield of (S)-N-(2-pyridyl)lactamide was 62.6%, with 21.5% of 2-aminopyridine remaining unreacted. This reaction mixture was concentrated under reduced pressure and following addition of 40 ml of water and 80 ml of ethyl acetate, it was adjusted to pH 0.8 with 13.5 ml of 1N-hydrochloric acid. After phase separation, the organic layer was discarded. The aqueous solution was adjusted to pH 4.5 with aqueous solution of NaOH and extracted twice with 50 ml of ethyl acetate (2-aminopyridine was retained in the aqueous layer). The organic layer was concentrated under reduced pressure to give 17.0 g of pale yellow solid. Analysis by HPLC revealed that the optical purity of this (S)-N-(2-pyridyl)lactamide was 93.0% ee.

The above product was dissolved in 75 ml of toluene under heating and allowed to stand at room temperature. The resulting crystal crop was collected by filtration to give (S)-N-(2-pyridyl)lactamide as white crystals (8.8 g). HPLC analysis revealed that the optical purity was 99.7% ee.

### Example 3 Production of (S)-N-(2-pyridyl)lactamide

(S)-Methyl lactate (optical purity = 98.9% ee) 28.4 g (272 mmol) and 128.2 g (136 mmol) of 2-aminopyridine were admixed and reacted under heating at 80°C for 5 days. Analysis of the reaction mixture at completion of the reaction by HPLC revealed that the yield of (S)-N-(2-pyridyl)lactamide was 52.5%, with 24.8% of 2-aminopyridine remaining unreacted. This reaction mixture was concentrated under reduced pressure, diluted with 40 ml of water, and extracted twice with 50 ml portions of ethyl acetate while the system was adjusted to pH 4.5 with concentrated hydrochloric acid (2-aminopyridine was retained in the aqueous layer). The organic layer was concentrated to dryness under reduced pressure to give 10.0 g of pale yellow solid. HPLC analysis revealed that the optical purity of (S)-N-(2-pyridyl)lactamide was 93.9% ee.

The above product was dissolved in 75 ml of toluene under heating and allowed to stand at room temperature but no separation of crystals occurred.

### Example 4 to 11 Production (S)-N-(2-pyridyl)lactamide

2-Aminopyridine 0.47 g (5 mmol) was admixed with (S)-methyl lactate (optical purity = 98.9% ee: 1 to 3 equivalents) and reacted under heating at the following temperature. The reaction mixture was sampled at a predetermined time and analyzed by the same procedure as in Example 1. The results are presented below in Table 1.

**Table 1**

| Example | Temperature (°C) | Methyl lactate (e q) | Time (h r s) | Conversion rate (%) | Optical purity (% e e) |
|---|---|---|---|---|---|
| 4 | 125 | 1 | 2 | 54.2 | 79.2 |
| 5 | 125 | 2 | 2 | 55.3 | 81.1 |
| 6 | 125 | 3 | 2 | 51.6 | 82.4 |
| 7 | 125 | 1 | 2 | 58.1 | 77.6 |
| 8 | 125 | 1 | 5 | 71.6 | 65.6 |
| 9 | 125 | 1 | 8 | 74.5 | 55.3 |
| 10 | 100 | 2 | 24 | 71.3 | 89.8 |
| 11 | 90 | 2 | 78 | 71.7 | 93.5 |

### Example 12 Production of (S)-N-(2-pyridyl)lactamide

(S)-Ethyl lactate (optical purity = 97% ee) 11.8 g (100 mmol) and 4.71 g (50 mmol) of 2-aminopyridine were dissolved in 50 ml of toluene and the solution was refluxed for 18 hours. The conversion rate was 6%.

### Example 13 Production of (S)-N-(2-pyridyl)lactamide

(S)-5-Methyl-2,2-dimethyl-1,3-dioxolan-4-one (optical purity = 97% ee) 260 mg (2 mmol) and 94 mg (1 mmol) of 2-aminopyridine were admixed and reacted under heating at 90°C for 14 hours. The conversion rate was 83.3% and the optical purity was 95.7% ee.

### Example 14 Production of (R)-N-(2-pyridyl)lactamide

(R)-Methyl lactate (optical purity = 96% ee) 20.82 g (200 mmol) and 9.41 g (100 mmol) of 2-aminopyridine were admixed and reacted under heating at 86°C for 5 days. After completion of the reaction, the reaction mixture was analyzed by HPLC. The conversion rate was 81%.

This reaction mixture was concentrated under reduced pressure to remove the residual (R)-methyl lactate to some extent and the concentrate was dissolved in 25 ml of 1N-hydrochloric acid. Then, 25 mL of ethyl acetate was added, and after phase separation, the organic layer was discarded (pH ≤ 1). The aqueous layer was adjusted to pH 4.5 with aqueous solution of NaOH and extracted twice with 50 ml portions of ethyl acetate (2-aminopyridine remained in the aqueous layer). The organic layer was concentrated under reduced pressure to give 12.6 g of pale yellow solid.

A portion of the above product was purified by silica gel chromatography to give (R)-N-(2-pyridyl)lactamide as white crystals (45 mg) . As analyzed by HPLC, the optical purity was 95.1% ee.
¹H-NMR (CDCl₃, 400 MHz/ppm): 1.57 (3H, d), 4.44 (1H, q), 5.39 (1H, bs), 7.09 (1H, dd), 7.75 (1H, dd), 8.25 (1H, d), 8.30 (1H, d), 9.48 (1H, bs).

### Example 15 Isolation and purification of (S)-N-(2-pyridyl)lactamide

In 6 ml of ethyl acetate was dissolved 6.1 g of the (S)-N- (2-pyridyl) lactamide obtained in Example 1 under heating and the solution was cooled to room temperature. Then, 18 ml of hexane was added dropwise and the mixture was stirred for 1 hour. The resulting crystals were collected by filtration, washed with hexane, and dried in vacuo to give 4 g of white crystals. The optical purity was 96.5% ee.

### Example 16 Isolation and purification of (S)-N-(2-pyridyl) lactamide

A prepared concentrate of (S)-N-(2-pyridyl)lactamide (optical'purity = 95.7% ee) 10.3 g was dissolved in 50 ml of toluene under heating, and after cooling to room temperature, the solution was stirred for 1 hour. The resulting crystals were collected by filtration, washed with hexane, and dried in vacuo to give 4.8 g of white crystals. The optical purity of the crystals was 99.7% ee.
[α]_{D}²⁵ = -25.63° (c = 1.034)
¹H-NMR (CDCl₃, 400 MHz/ppm): 1.57 (3H, d) , 4.44 (1H, q) , 5.39 (1H, bs), 7.09 (1H, dd), 7.75 (1H, dd), 8.25 (1H, d), 8.30 (1H, d), 9.48 (1H, bs). mp = 102 to 104°C

### Example 17 Isolation and purification of (S)-N-(2-pyridyl)lactamide

In 15 ml of ethylbenzene was dissolved 3.00 g of (S)-N-(2-pyridyl)lactamide (optical purity = 90.5% ee) under heating, and after cooling to room temperature, the solution was stirred for 1 hour. The resulting crystals were collected by filtration, washed with hexane, and dried in vacuo to give 2.44 g of white crystals. The optical purity of the crystals was 99.0% ee.

### Example 18 Isolation and purification of (S)-N-(2-pyridyl)lactamide

In 15 ml of isopropyl alcohol was dissolved 3.00 g of (S)-N-(2-pyridyl)lactamide (optical purity = 90.0% ee) under heating, and after cooling to -5°C, the solution was stirred for 1 hour. The resulting crystals were collected by filtration, washed with hexane, and dried in vacuo to give 1.30 g of white crystals. The optical purity of the crystals was 95.4% ee.

### Example 19 Production of (S)-N-(2-pyridyl)-1-amino-2-propanol

In 10 ml THF-10 ml toluene was dissolved 3 g (20 mmol) of the (S)-N-(2-pyridyl)lactamide obtained in Example 16, and the solution was added dropwise to a suspension of 1.14 g (30 mmol) of lithium aluminum hydride in 5 ml of THF, followed by 1 hour of stirring. The mixture was heated to 80°C and stirred for an additional 1 hour and 30 ml of water was added. This reaction mixture was extracted 3 times with 60 ml portions of ethyl acetate. The organic layers were combined, dried over anhydrous sodium sulfate, and concentrated under reduced pressure to give (S)-N-(2-pyridyl)-1-amino-2-propanol as yellow oil (2.8 g). A 1.5 g portion of the above oil was distilled under reduced pressure to give (S)-N-(2-pyridyl)-1-amino-2-propanol as pale yellow oil (1.43 g) .

### Example 20 Production of (S)-N-(2-pyridyl)-1-amino-2-propanol

In 40 ml of THF was suspended 11.6 g (300 mmol) of sodium borohydride, and a THF solution of 16.7 g (100 mmol) of (S)-N-(2-pyridyl)lactamide was added dropwise under ice-cooling. After completion of dropwise addition, a solution of 15.2 g of sulfuric acid in 80 ml of THF was added dropwise while the system was maintained at an internal temperature of not over 10°C. After completion of dropwise addition, the internal temperature was increased to room temperature and the mixture was then stirred under reflux for 2 hours.

Separately, 20.9 g (200 mmol) of concentrated HCl was mixed with 200 ml of water and the above reaction mixture was continuously added to this mixture. After completion of addition, the whole mixture was warmed to 50°C and stirred for 2 hours, at the end of which time THF was distilled off. To 255.2 g of the aqueous solution thus obtained was added 200 ml of ethyl acetate, and after the mixture was adjusted to pH 9.4 with 59.9 g of 30% aqueous solution of NaOH, the product was extracted into the organic layer. The aqueous layer was further extracted with 200 ml of ethyl acetate and the extracts were combined and washed with 10 ml of water. The organic layer was distilled under reduced pressure to give 15.9 g of crude (S)-N-(2-pyridyl)-1-amino-2-propanol. A 13.8 g portion of this product was purified by distillation under reduced pressure to give 9.7 g of (S)-N-(2-pyridyl)-1-amino-2-propanol as a pure product.

### Example 21 Treatment with water

An ethyl acetate extract of (S)-N-(2-pyridyl)-1-amino-2-propanol as prepared in the same manner as in Example 20 was treated with water and the distillation recovery rate and residual rate in residue were determined and compared with the corresponding values found without the treatment with water. The results are shown below in Table 2.

**Table 2**

| Treatment * with water | Distillation recovery rate | Residual rate |
|---|---|---|
| Yes | 96 % | 3 % |
| No | 75 % | 22% |

| | | |
|---|---|---|
| * : "Treatment with waters means" washing the extract with 5 ml of water per 196 g. | | |

### Example 22 Treatment with an alcohol

A concentrate of (S)-N-(2-pyridyl)-1-amino-2-propanol as prepared in the same manner as in Example 20 was treated with methanol and the distillation recovery rate and residual rate in residue were determined and compared with the corresponding values found without the treatment with methanol. The results are shown below in Table 3.

**Table 3**

| Treatment with metanol* | Distillation recovery rate | Residual rate Residual rate |
|---|---|---|
| Yes | 87 % | 7 % |
| No | 75 % | 22 % |

| | | |
|---|---|---|
| *: "Treatment with metanol" means a procedure which comprises adding methanol to the concentrate and subjecting the mixture to distillation under reduced pressure. (In Example 22, methanol was added at a ratio of 40 ml per 20 g concentrate and subjecting the mixture to distillation under reduced pressure in two repeats. ) | | |

### Example 23 Distillation of (S)-N-(2-pyridyl)-1-amino-2-propanol

In the case (Case 1) where, in the distillation of (S)-N-(2-pyridyl)-l-amino-2-propanol, (S)-N-(2-pyridyl)-1-amino-2-propanol and contained 2-aminopyridine are to be distilled (recovered) independently from different distillate lines, the case (Case 2) in which the condenser is washed after distillation of 2-aminopyridine and (S)-N-(2-pyridyl)-1-amino-2-propanol is then distilled (recovered), the case (Case 3) in which the two compounds are distilled together (recovered) from one and the same distillate line without the above treatment, and the case (case 4) in which both compounds are distilled (recovered) from one and the same distillate line with the condenser temperature being fixed at 70°C, the percent loss of (S)-N-(2-pyridyl)-1-amino-2-propanol upon the first drop off, the main fraction recovery rate, and the 2-aminopyridine content of the main distillate were determined for inter-comparison. The respective procedures and results were as follows.
Case 1: 102.1 g of (S)-N-(2-pyridyl)-1-amino-2-propanol containing 2-aminopyridine (4.8 area %/HPLC) was distilled under reduced pressure to give a first drop of 6.8 g in the first place (said loss rate: 3%). Then, 90.3 g of a main fraction was obtained through another distillate line (degree of vacuum = ca. 3 mmHg, distillation temperature = 115 to 120°C). The main fraction recovery rate was 93% and the 2-aminopyridine content of the main fraction was 0.7 area %/HPLC.
Case 2: 105.3 g of (S)-N-(2-pyridyl)-1-amino-2-propanol containing 2-aminopyridine (4.8 area %/HPLC) was distilled under reduced pressure to give a first drop of 7.3 g in the first place (said loss rate: 3%). Then, after washing the distillate line, 89.2 g of a main fraction was obtained through the same distillate line (degree of vacuum = ca. 3 mmHg, distillation temperature = 115 to 120°C). The main fraction recovery rate was 92% and the 2-aminopyridine content of the main fraction was 0.6 area %/HPLC.
Case 3: 80.1 g of (S)-N-(2-pyridyl)-1-amino-2-propanol containing 2-aminopyridine (4.8 area %/HPLC) was distilled under reduced pressure to give a first drop of 12.4 g in the first place (said loss rate: 14%). Then, a main fraction of 62.5 g was obtained from the same distillate line (degree of vacuum = ca. 3mmHg/distillation temperature = 115 to 120°C). The main fraction recovery rate was 82% and the 2-aminopyridine content of the main fraction was 2.8 area %/HPLC.
Case 4: 101.7 g of (S)-N-(2-pyridyl)-1-amino-2-propanol containing 2-aminopyridine (4.9 area %/HPLC) was distilled under reduced pressure and a first drop of 7.5 g was obtained from the distillate line (inclusive of the condenser) controlled at 70°C with a ribbon heater (said loss rate = 4%). Then, through the same distillate line, 88.5 g of a main fraction was obtained (degree of vacuum = ca. 3 mmHg/distillation temperature = 115 to 120°C). The main fraction recovery rate was 91% and the 2-aminopyridine content of the main fraction was 0.8 area %/HPLC.

Table 4 below shows the loss rates of (S)-N-(2-pyridyl)-1-amino-2-propanol upon the first drop off, main fraction recovery rates, and 2-aminopyridine contents of main fractions in the respective cases.

**Table 4**

| Case Case | ^{* 1} Loss rate | Main fraction recovery rate | Content^{*2} % Content |
|---|---|---|---|
| 1 | 3 % | 93 % | 0.7 % |
| 2 | 3% | 92% | 0.6% |
| 3 | 14% | 82% | 2.8% |
| 4 | 4 % | 91 % | 0.8% |

| | | | |
|---|---|---|---|
| *1 : Loss rate means the percent loss of (S)-N-(2-pyridyl)-1-amino-2-propanol upon the first drop off. | | | |
| *2 : % Content means the 2-aminopyridine content (area %/HPLC) of the main fraction. | | | |

### INDUSTRIAL APPLICABILITY

In accordance with the process for the invention, optically active N-aryl-1-amino-2-propanol derivatives of value as pharmaceutical intermediates can be expediently produced from inexpensive starting compounds.

## Claims

1. An optically active N-pyridyllactamide derivative which is represented by the general formula (4a): 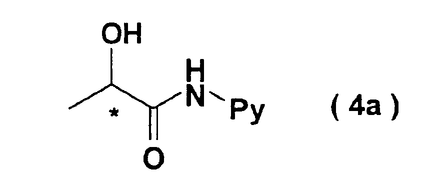 in the formula, Py represents 2-pyridyl group which may be substituted, 3-pyridyl group which may be substituted, or 4-pyridyl group which may be substituted; * represents an asymmetric carbon atom.

2. The optically active N-pyridyllactamide derivative according to Claim 1, which is crystalline.

3. The optically active N-pyridyllactamide derivative according to Claim 1 or 2,
wherein the substituent or substituents are at least one member selected from the group consisting of halogens, and nitro, N-protected amino, C₁₋₁₀ alkyl, and C₁₋₁₀ alkyloxy groups.

4. The optically active N-pyridyllactamide derivative according to any one of Claims 1 to 3,
wherein Py is 2-pyridyl group.

5. The optically active N-pyridyllactamide derivative according to any one of Claims 1 to 4,
the absolute configuration of which is S.

6. A process for producing an optically active N-aryllactamide derivative of the general formula (4): 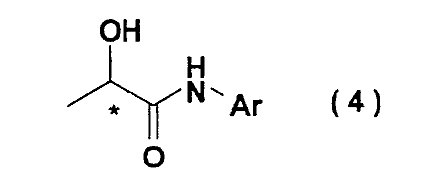 in the formula, Ar represents an aromatic group which may be substituted; * represents an asymmetric carbon atom,
which comprises reacting an optically active lactate derivative of the general formula (1): 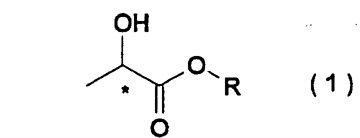 in the formula, R represents a C₁₋₁₀ alkyl group; * is as defined above
or an optically active lactic acid acetal derivative of the general formula (2): 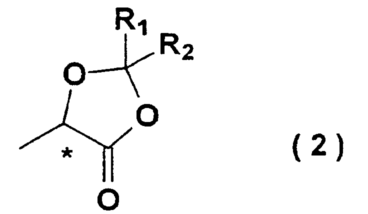 in the formula, R₁ and R₂ each independently represents hydrogen atom, a C₁₋₁₀ alkyloxy group, or a C₁₋₁₀ alkyl group; * is as defined above
with an arylamine derivative of the general formula (3):
Ar-NH₂ (3)
in the formula, Ar is as defined above.

7. The process according to Claim 6,
wherein the optically active lactate derivative of the general formula (1) is used.

8. The process according to Claim 6 or 7,
wherein R in the general formula (1) is methyl or ethyl group.

9. The process according to Claim 6,
wherein both of R₁ and R₂ in the general formula (2) are methyl groups.

10. The process according to any one of Claims 6 to 9,
wherein Ar is 2-pyridyl group which may be substituted, 3-pyridyl-group which may be substituted, 4-pyridyl group which may be substituted, or phenyl group which may be substituted.

11. The process according to any one of Claims 6 to 10,
wherein the substituent or substituents are at least one member selected from the group consisting of halogens, and nitro, N-protected amino, C₁₋₁₀ alkyl and C₁₋₁₀ alkyloxy groups.

12. The process according to Claim 10 or 11,
wherein Ar is 2-pyridyl group.

13. The process according to any one of Claims 6 to 12,
wherein the molar ratio of said optically active lactate derivative (1) or optically active lactic acid acetal derivative (2) to said arylamine derivative (3) is 1:1 to 3:1.

14. The process according to any one of Claims 6 to 13,
wherein the level of use of a reaction solvent is not more than 10 times weight of the arylamine derivative (3).

15. The process according to Claim 14,
wherein the reaction is carried out in the absence of a reaction solvent.

16. The process according to any one of Claims 6 to 15,
wherein the reaction temperature is 70°C to 130°C.

17. A process for purifying an optically active N-pyridyllactamide derivative,
which comprises fractionating an optically active N-pyridyllactamide derivative of the general formula (4a): 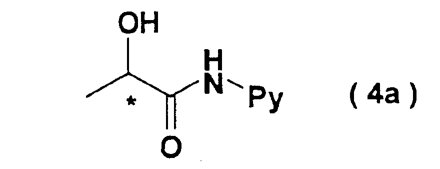 in the formula, Py represents 2-pyridyl group which may be substituted, 3-pyridyl group which may be substituted, or 4-pyridyl group which may be substituted; * represents an asymmetric carbon atom
containing an optically active lactate derivative of the general formula (1): 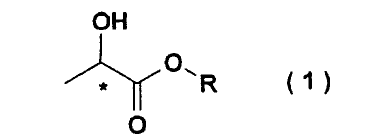 in the formula, R represents a C₁₋₁₀ alkyl group; * is as defined above
or an optically active lactic acid acetal derivative of the general formula (2) : 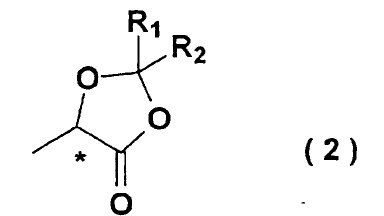 in the formula, R₁ and R₂ each independently represents hydrogen atom, a C₁₋₁₀ alkyloxy group, or a C₁₋₁₀ alkyl group; * is as defined above
and any byproduct derived therefrom as impurities in a biphasic system comprising water and an organic solvent under an acidic condition with the optically active N-pyridyllactamide derivative (4a) being separated in an aqueous layer and
removing the organic solvent layer.

18. The process according to Claim 17,
wherein the organic solvent is an ester.

19. The process according to Claim 17 or 18,
wherein the acidic condition is not over pH 2.

20. The process according to any one of Claims 17 to 19,
wherein the optically active N-pyridyllactamide derivative (4a) is one obtainable by the process according to any one of Claims 10 to 16.

21. A process for purifying an optically active N-pyridyllactamide derivative,
which comprises purifying an optically active N-pyridyllactamide derivative of the general formula (4a): 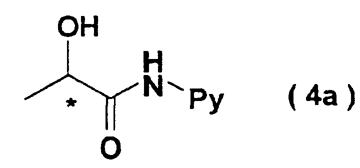 in the formula, Py is as defined above; * represents an asymmetric carbon atom
containing an aminopyridine derivative of the general formula (3a):
Py-NH₂ (3a)
in the formula, Py represents 2-pyridyl group which may be substituted, 3-pyridyl group which may be substituted, or 4-pyridyl group which may be substituted
by extracting the optically active N-pyridyllactamide derivative (4a) into an organic layer of a biphasic system comprising water and an organic solvent under a weakly acidic condition with the aminopyridine derivative (3a) being retained in the aqueous layer.

22. The process according to Claim 21,
wherein the organic solvent is an ester.

23. The process according to Claim 21 or 22,
wherein the weakly acidic condition is pH 3 to 6.

24. The process according to any one of Claims 21 to 23,
wherein the optically active N-pyridyllactamide derivative (4a) is one obtainable by the process according to any one of Claims 10 to 20.

25. A process for purifying an optically active N-pyridyllactamide derivative,
which comprises crystallizing an optically active N-pyridyllactamide derivative of the general formula (4a): 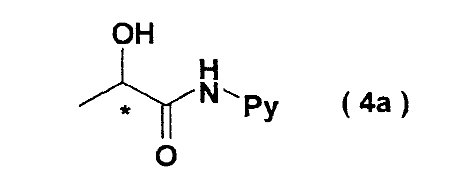 in the formula, Py represents 2-pyridyl group which may be substituted, 3-pyridyl group which may be substituted, or 4-pyridyl group which may be substituted; * represents an asymmetric carbon atom
containing an impurity from an organic solvent to thereby remove the impurity and obtain the N-pyridyllactamide derivative (4a) as a crystal.

26. The process according to Claim 25,
wherein the impurity contained in the optically active N-pyridyllactamide derivative of the formula (4a) is the enantiomer of the compound of the formula (4a).

27. The process according to Claim 25 or 26,
wherein the organic solvent is at least one member selected from the group consisting of aromatic hydrocarbons, alcohols, ethers, halogen-containing solvents, esters, ketones, nitrogen-containing solvents, and aprotic polar solvents.

28. The process according to Claim 27,
wherein the organic solvent is an aromatic hydrocarbon.

29. The process according to Claim 28,
wherein the aromatic hydrocarbon is toluene.

30. The process according to Claim 28 or 29,
wherein an auxiliary solvent is used for improving at least one of yield, treating concentration, and slurry fluidity in the crystallization, of the compound of the formula (4a) and purity and physical properties of the resulting crystal.

31. The process according to Claim 30,
wherein the auxiliary solvent is at least one member selected from the group consisting of aliphatic hydrocarbons, esters, and amines.

32. The process according to any one of Claims 25 to 31,
wherein the crystallization is carried out by utilizing at least one of cooling crystallization, concentrating crystallization, and neutralizing crystallization.

33. The process according to any one of Claims 25 to 32,
wherein the crystallization is carried out by utilizing cooling crystallization or in combination of cooling crystallization with concentrating crystallization, or neutralizing crystallization.

34. The process according to any one of Claims 25 to 33,
wherein the optically active N-pyridyllactamide derivative (4a) obtainable by the process according to any one of Claims 10 to 24 is used.

35. A process for producing an optically active N-aryl-1-amino-2-propanol derivative of the general formula (5) : 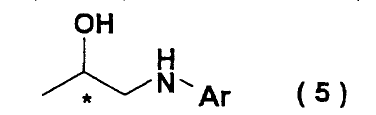 in the formula, Ar represents an aromatic group which may substituted; * represents an asymmetric carbon atom,
which comprises reacting either an optically active lactate derivative of the general formula (1): 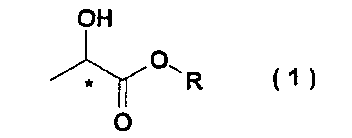 in the formula, R represents a C₁₋₁₀ alkyl group; * is as defined above
or an optically active lactic acid acetal compound of the general formula (2): 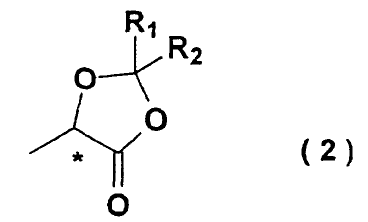 in the formula, R₁ and R₂ each independently represents hydrogen atom, a C₁₋₁₀alkyloxy group, or a C₁₋₁₀ alkyl group; * is as defined above
with an arylamine derivative of the general formula (3):
Ar-NH₂ (3)
in the formula, Ar is as defined above
to give an optically active N-aryllactamide derivative of the general formula (4): 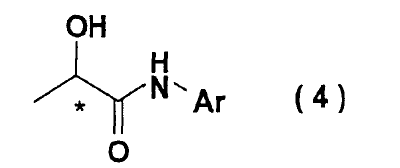 in the formula, Ar and * are as defined above and
treating the derivative with a reducing agent.

36. The process according to Claim 35,
wherein an optically active lactate derivative of the general formula (1) is used.

37. The process according to Claim 35 or 36,
wherein R in the general formula (1) is methyl or ethyl group.

38. The process according to Claim 35,
wherein both of R₁ and R₂ in the general formula (2) are methyl groups.

39. The process according to any one of Claims 35 to 38,
wherein Ar is 2-pyridyl group which may be substituted, 3-pyridyl group which may be substituted, 4-pyridyl group which may be substituted, or phenyl group which may be substituted.

40. The process according to any one of Claims 35 to 39,
wherein the substituent or substituents are at least one member selected from the group consisting of halogens, and nitro, N-protected amino, C₁₋₁₀ alkyl and C₁₋₁₀ alkyloxy groups.

41. The process according to Claim 39,
wherein Ar is 2-pyridyl group.

42. The process according to any one of Claims 35 to 41,
wherein the molar ratio of said optically active lactate derivative (1) or optically active lactic acid acetal derivative (2) to said arylamine derivative (3) is 1:1 to 3:1.

43. The process according to any one of Claims 35 to 42,
wherein the level of use of a reaction solvent for the reaction of said optically active lactate derivative (1) or optically active lactic acid acetal derivative (2) with said allylamine derivative (3) is not more than 10 times weight of the arylamine derivative (3).

44. The process according to Claim 43,
wherein the reaction is carried out in the absence of a reaction solvent.

45. The process according to any one of Claims 35 to 44,
wherein the reaction temperature for the reaction of said optically active lactate derivative (1) or optically active lactic acid acetal derivative (2) with said arylamine derivative (3) is 70°C to 130°C.

46. The process according to any one of Claims 35 to 45,
wherein the reducing agent is a borane derivative, an aluminum hydride complex compound, or a boron hydride complex compound.

47. The process according to Claim 46,
wherein the borane derivative is a borane or a borane-THF complex.

48. The process according to Claim 46,
wherein the aluminum hydride complex compound is lithium aluminum hydride.

49. The process according to Claim 46,
wherein the boron hydride complex compound is sodium borohydride or potassium borohydride.

50. The process according to Claim 46 or 47,
wherein the borane derivative is one generated by treating a boron hydride complex compound with an acid.

51. The process according to Claim 50,
wherein the boron hydride complex compound is sodium borohydride or potassium borohydride.

52. The process according to any one of Claims 35 to 51,
wherein the reaction solvent for a reduction reaction is an ether.

53. The process according to Claim 52,
wherein the ether is a cyclic ether.

54. A process for producing an optically active N-aryl-1-amino-2-propanol derivative of the general formula (5): 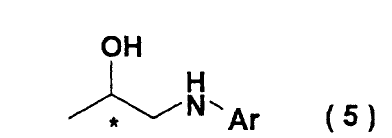 in the formula, Ar represents an aromatic group which may be substituted; * represents an asymmetric carbon atom,
which comprises reducing an optically active N-aryllactamide derivative of the general formula (4): 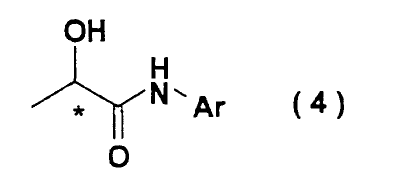 in the formula, Ar and * are as defined above
with a borane derivative generated by treating a boron hydride complex compound with an acid.

55. The process according to Claim 54,
wherein Ar is 2-pyridyl group which may be substituted, 3-pyridyl group which may be substituted, 4-pyridyl group which may be substituted, or phenyl group which may be substituted.

56. The process according to Claim 54 or 55,
wherein Ar is 2-pyridyl group.

57. The process according to any one of Claims 54 to 56,
wherein the boron hydride complex compound is sodium borohydride or potassium borohydride.

58. The process according to any one of Claims 54 to 57,
wherein the reaction solvent is an ether.

59. A process for acquiring an optically active N-aryl-1-amino-2-propanol derivative,
which comprises treating, under an acidic condition, boron-coordinated complex of an optically active N-aryl-1-amino-2-propanol derivative occurring in an optically active N-aryl-1-amino-2-propanol derivative of the general formula (5): 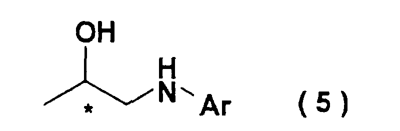 in the formula, Ar represents an aromatic group which may be substituted; * represents an asymmetric carbon atom
obtainable by reducing an optically active N-aryllactamide derivative of the general formula (4): 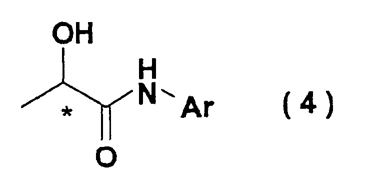 in the formula, Ar and * are as defined above
with a borane derivative or a boron hydride complex compound, to thereby give the optically active N-aryl-1-amino-2-propanol derivative (5).

60. The process according to Claim 59,
wherein the acidic condition is not over pH 3.

61. A process for acquiring an optically active N-aryl-1-amino-2-propanol derivative,
which comprises fractionating the optically active N-aryl-1-amino-2-propanol derivative (5) obtainable by the process according to Claim 59 or 60 in a biphasic system comprising water and an organic solvent under a weakly acidic to basic condition to acquire an organic layer containing the optically active N-aryl-1-amino-2-propanol derivative (5).

62. The process according to Claim 61,
wherein the weakly acidic to basic condition is pH 5 to 13.

63. The process according to any one of Claims 59 to 62,
wherein the optically active N-aryl-1-amino-2-propanol derivative (5) is one obtainable by the process according to any one of Claims 46 to 58.

64. The process according to any one of Claims 59 to 63,
wherein Ar is 2-pyridyl group which may be substituted, 3-pyridyl group which may be substituted, 4-pyridyl group which may be substituted, or phenyl group which may be substituted.

65. The process according to Claim 64,
wherein Ar is 2-pyridyl group.

66. A process for acquiring an optically active N-aryl-1-amino-2-propanol derivative by distillation of an optically active N-aryl-1-amino-2-propanol derivative of the general formula (5): 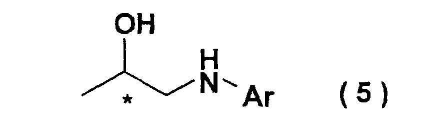 in the formula, Ar represents an aromatic group which may be substituted; * represents an asymmetric carbon atom
containing an arylamine derivative of the general formula (3) :
Ar-NH₂ (3)
in the formula, Ar is as defined above
as an impurity,
which comprises distilling and recovering the arylamine derivative (3) and, then, distilling and recovering the optically active N-aryl-1-amino-2-propanol derivative (5) from a distillate line inclusive of a condenser which is essentially not contaminated with the arylamine derivative (3).

67. The process according to Claim 66,
wherein the distillation is carried out under reduced pressure.

68. The process according to Claim 66 or 67,
wherein the optically active N-aryl-1-amino-2-propanol derivative (5) obtainable by the process according to any one of Claims 35 to 65 is used.

69. The process according to any one of Claims 66 to 68,
which comprises, in acquiring an optically active N-aryl-1-amino-2-propanol derivative (5) by distillation, treating a boron component-contaminated extract or concentrate of the optically active N-aryl-1-amino-2-propanol derivative (5) with water and/or an alcohol for decrease or removal of the boron component.

70. The process according to Claim 69,
wherein the alcohol is a monohydric alcohol containing 1 to 3 carbon atoms.

71. The process according to Claim 69 or 70,
wherein the boron component is removed or decreased, by treating with water and/or the alcohol, to a level of not more than 10 mole % relative to the optically active N-aryl-1-amino-2-propanol derivative (5).

72. The process according to any one of Claims 66 to 71,
wherein Ar is 2-pyridyl group which may be substituted, 3-pyridyl group which may be substituted, 4-pyridyl group which may be substituted, or phenyl group which may be substituted.

73. The process according to any one of Claims 66 to 72,
wherein Ar is 2-pyridyl group.

74. A process for acquiring an optically active N-aryl-1-amino-2-propanol derivative of the general formula (5): 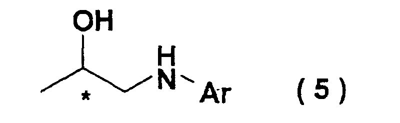 in the formula, Ar represents an aromatic group which may be substituted; * represents an asymmetric carbon atom
by distillation,
which comprises treating a boron component-contaminated extract or concentrate of the optically active N-aryl-1-amino-2-propanol derivative (5) with water and/or an alcohol for decrease or removal of the boron component.

75. The process according to Claim 74,
wherein the alcohol is a monohydric alcohol of 1 to 3 carbon atoms.

76. The process according to Claim 74 or 75,
wherein the boron component is removed or decreased, by treating with water and/or the alcohol, to a level not over 10 mole % relative to the optically active N-aryl-1-amino-2-propanol derivative (5).

77. The process according to any one of Claims 74 to 76,
wherein the optically active N-aryl-1-amino-2-propanol derivative (5) obtainable by the process according to any one of Claims 46 to 65 is used.

78. The process according to any one of Claims 74 to 77,
wherein Ar is 2-pyridyl group which may be substituted, 3-pyridyl group which may be substituted, 4-pyridyl group which may be substituted, or phenyl group which may be substituted.

79. The process according to any one of Claims 74 to 78,
wherein Ar is 2-pyridyl group.
